# EUROPEAN PATENT APPLICATION

(11) **EP 2 191 860 A1**
(43) Date of publication of application: **02.06.2010**
(21) Application number: 09177007.3
(22) Date of filing: 25.11.2009
(51) Int. Cl.: A61M 5/28, A61M 5/31

(54) **Combined container-syringe**

(30) Priority: 28.11.2008 JP 2008304608
(71) Applicant: ARTE CORPORATION, Tokyo (JP)
(72) Inventor: Kakiuchi, Makoto, Takahagi-shi Ibaraki (JP); Takeshima, Yasuhiko, Chiyoda-ku Tokyo (JP)
(74) Representative: Metman, Karel Johannes

(57) **Abstract**

A combined container-syringe includes: a cartridge (1); a needle hub (20) having a base portion (21), a cylinder portion (22) inside which a bypass chamber (25) is formed, and a needle attachment portion (23) inside which an introducing hole (23a) communicated with an injection needle (8) is formed; a front stopper (10); a rear stopper (5); and a plunger rod (6) connected to the rear stopper, in which: a bypass groove (26) communicated with the introducing hole, and an annular groove (27) connected to the bypass groove are formed on an inner circumferential surface of the bypass chamber; an air-bubble introducing groove (13) is formed on an outer circumferential surface of the front stopper, the air-bubble introducing groove extending from a rear end surface of the front stopper toward a front end; and the air-bubble introducing groove and the annular groove are communicated with each other in a state where a part of the front stopper is disposed in the cartridge.

## Description

### BACKGROUND OF THE INVENTION

### Field of the Invention

The present invention relates to a combined container-syringe which is prefilled with a drug solution which is stored therein, and which can be used immediately after being removed from the packaging at the time of use.

### Description of Related Art

A combined container-syringe can be used immediately after being removed from the packaging without the need for troublesome procedures to be preformed at medical institutions due to the fact that the combined container-syringe has been prefilled with the drug solution. Therefore, it is very convenient and useful in terms of reducing the workload on those people working in the medical industry, such as physicians and nurses. For this reason, it is being adopted by many medical facilities.

Examples of conventional combined container-syringes include a combined container-syringe 41 shown in FIG 9 (refer to Japanese Unexamined Patent Application, First Publication No. 2007-111156). The combined container-syringe 41 includes a glass cartridge 42, a front stopper 45a which is fitted in a front end portion of the glass cartridge 42, a rear stopper 45b which is fitted in a rear end portion of the glass cartridge 42, a hub-luer lock 43 which is fitted on an outer circumferential surface of the front end portion of the glass cartridge 42, a finger grip 44 which is fitted on the rear end portion of the glass cartridge 42, and a plunger rod 46 which is inserted into the glass cartridge 42 from the rear end of the glass cartridge 42 and is connected to the rear stopper 45b. The front stopper 45a and the rear stopper 45b seal the drug solution m stored in the glass cartridge 42 in a liquid-tight manner. The hub-luer lock 43 has a bypass chamber 48 into which the front stopper 45a is inserted, and a luer tip 49 which is provided to a front end portion of the bypass chamber 48 and is used for attaching an injection needle.

When using this combined container-syringe 41, the rear stopper 45b is pushed by the plunger rod 46 so that the drug solution and the front stopper 45a are moved forward. When the front stopper 45a is pushed out from the glass cartridge 42 and is inserted into the bypass chamber 48 of the hub-luer lock 43, the tight-sealing of the front end side of the glass cartridge 42 which is filled with the drug solution m, i.e., the sealing with the front stopper 45a, is released. As a result, the drug solution m flows from the glass cartridge 42 into the bypass chamber 48, flows in a gap between the front stopper 45a and the bypass chamber 48, is guided to the inside of the luer tip 49 via a bypass groove 48a provided on an inner surface of the bypass chamber 48, and is introduced into the injection needle.

Here, the injection is a medical procedure of directly injecting a drug solution to a desired site inside a patient's body with a syringe. In order to prevent air bubbles in a drug solution from being injected inside the patient's body, an air-eliminating step is performed before the injection by slightly pushing the plunger rod. With this step, air bubbles in the glass cartridge of the combined container-syringe pass through the bypass groove to be eliminated outside of the combined container-syringe via the front end of the injection needle.

However, in the combined container-syringe disclosed in Japanese Unexamined Patent Application, First Publication No. 2007-111156, the tight-sealing of the glass cartridge, which is filled with the drug solution in air-tight and liquid-tight manners, is released and the drug solution flows out toward the injection needle only by moving the front stopper forward in the glass cartridge to insert it into the bypass chamber. In the combined container-syringe disclosed in Japanese Unexamined Patent Application, First Publication No. 2007-111156, it is difficult because of surface tension for tiny air bubbles entering the side surface of the front stopper, the groove in the bypass chamber, and the like, to overtake the drug solution and move to the injection needle before the drug solution reaches the injection needle.

The present invention was devised in view of the above circumstances, and has as an object the provision of a combined container-syringe which is capable of reliably eliminating air bubbles in the drug solution via the front end of the injection needle without being affected by surface tension.

### SUMMARY OF THE INVENTION

A combined container-syringe according to an aspect of the present invention includes: a cartridge; a needle hub having a base portion which is fitted on an outer circumferential surface of a front end portion of the cartridge, a cylinder portion which is connected to a front end of the base portion and inside which a bypass chamber is formed, and a needle attachment portion which is connected to a front end of the cylinder portion and inside which an introducing hole communicated with an injection needle is formed; a front stopper which is fitted in the front end portion of the cartridge in a liquid-tight manner; a rear stopper which is fitted in a rear end portion of the cartridge in a liquid-tight manner; and a plunger rod which is connected to the rear stopper, in which: a bypass groove which extends along an axis of the cartridge and is communicated with the introducing hole, and an annular groove which is connected to a rear end of the bypass groove are formed on an inner circumferential surface of the bypass chamber; an air-bubble introducing groove is formed on an outer circumferential surface of the front stopper, the air-bubble introducing groove extending from a rear end surface of the front stopper toward a front end; and the air-bubble introducing groove and the annular groove are communicated with each other in a state where a part of the front stopper is disposed in the cartridge.

In the combined container-syringe according to the aspect of the present invention, the air-bubble introducing groove may extend, on the rear end surface of the front stopper, from an outer circumference of the rear end surface of the front stopper toward the axis of the cartridge.

In the combined container-syringe according to the aspect of the present invention, a plurality of the bypass grooves may be formed on the inner circumferential surface of the bypass chamber such that the bypass grooves are uniformly spaced in a circumferential direction of the inner circumferential surface of the bypass chamber; and a plurality of the air-bubble introducing grooves may be formed on the outer circumferential surface of the front stopper such that the air-bubble introducing grooves are uniformly spaced in a circumferential direction of the outer circumferential surface of the front stopper.

In the combined container-syringe according to the aspect of the present invention, the positions of the plurality of the air-bubble introducing grooves in the circumferential direction may correspond to the positions of the plurality of the bypass grooves.

According to the combined container-syringe of the present invention, the air-bubble introducing groove and the annular groove are communicated with each other in a state where a part of the front stopper is disposed in the cartridge. In this state, the air-bubble introducing groove is communicated with the annular groove, the bypass groove, and the introducing hole. Therefore, it is possible to effectively eliminate air bubbles which remain on the rear end surface of the front stopper by performing an air-eliminating step to move the air bubbles from the air-bubble introducing groove to the introducing hole.

Furthermore, according to the combined container-syringe of the present invention, the air-bubble introducing groove formed on the outer circumferential surface of the front stopper extends, on the rear end surface of the front stopper, from the outer circumference of the rear end surface of the front stopper toward the axis. Accordingly, it is possible to easily introduce air bubbles which remain on the rear end surface of the front stopper to the inside of the air-bubble introducing groove by performing an air-eliminating step. When pushing the plunger rod so as to insert the front end side portion of the front stopper into the bypass chamber, air bubbles introduced to the air-bubble introducing groove pass through the annular groove, the bypass groove, and the introducing hole to be eliminated outside of the combined container-syringe via the front end of the needle attachment portion. Therefore, it is possible to effectively eliminate air bubbles in the drug solution by performing an air-eliminating step.

### BRIEF DESCRIPTION OF THE DRAWINGS

FIG. 1 is a sectional side view showing a combined container-syringe according to one embodiment of the present invention in a state where the combined container-syringe is unused.
FIG 2 is an enlarged view in the vicinity of a front end portion of a glass cartridge of the combined container-syringe shown in FIG 1.
FIG. 3 is a side view showing a front stopper.
FIG 4 is a rear view showing the front stopper.
FIG. 5 is a sectional side view showing the combined container-syringe at the time of an air-eliminating step.
FIG 6 is an enlarged view in the vicinity of the front end portion of the glass cartridge of the combined container-syringe shown in FIG. 5.
FIG. 7 is a sectional side view showing the combined container-syringe after the completion of injection.
FIG 8 is an enlarged view in the vicinity of the front end portion of the glass cartridge of the combined container-syringe shown in FIG 7.

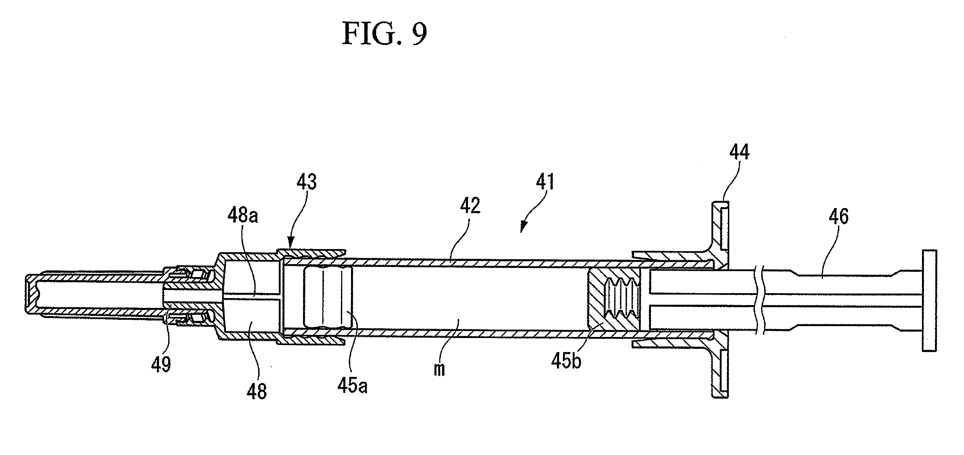

FIG. 9 is a sectional side view showing a conventional combined container-syringe.

### DETAILED DESCRIPTION OF THE INVENTION

An embodiment of a combined container-syringe according to the present invention will be described hereinbelow with reference to drawings.

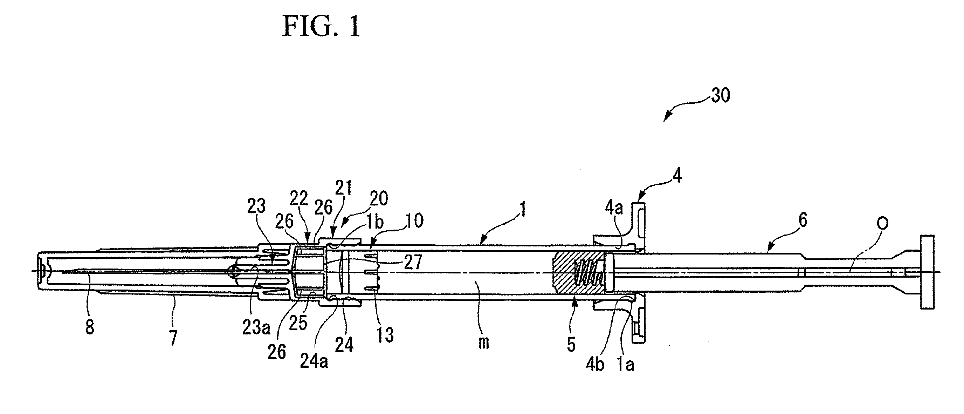

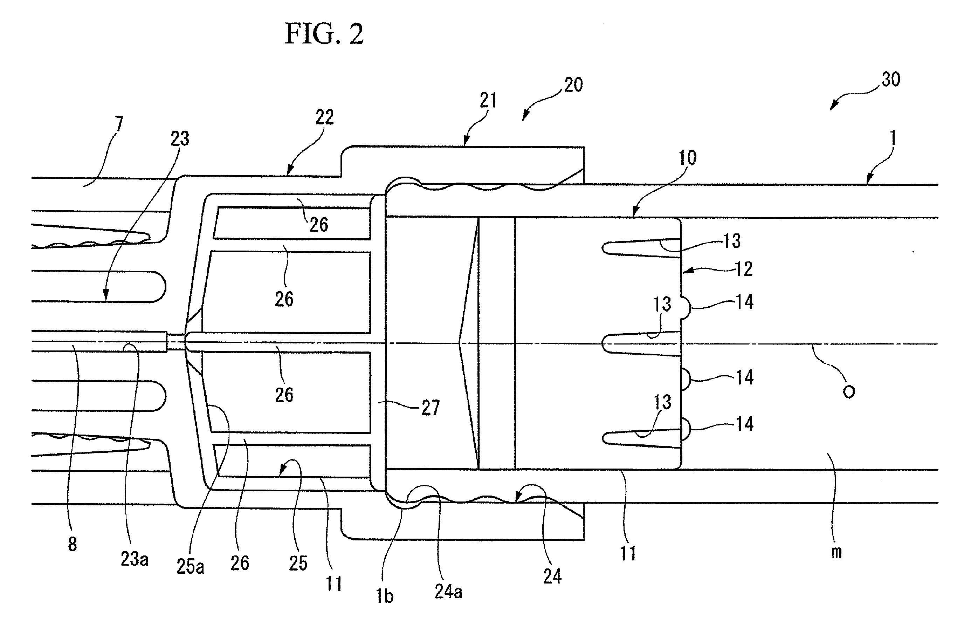

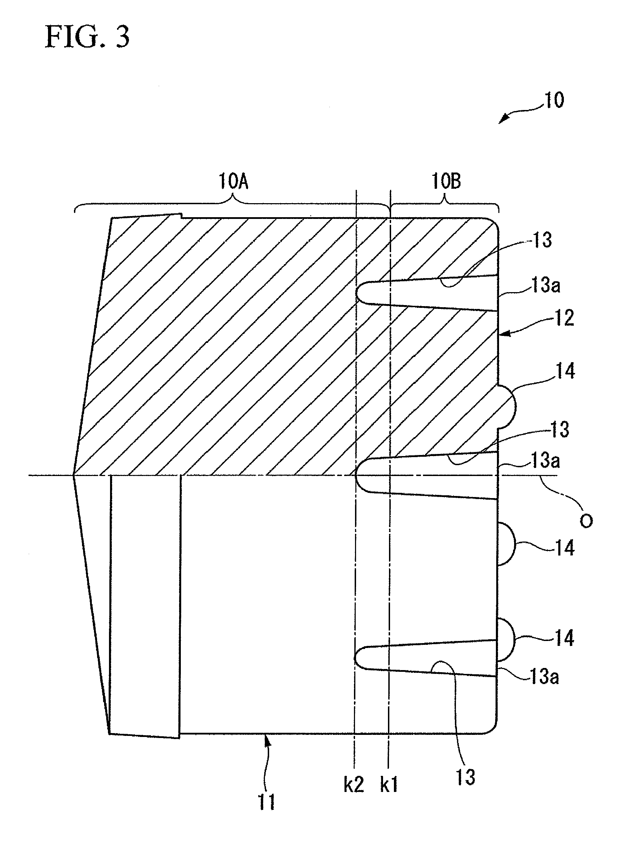

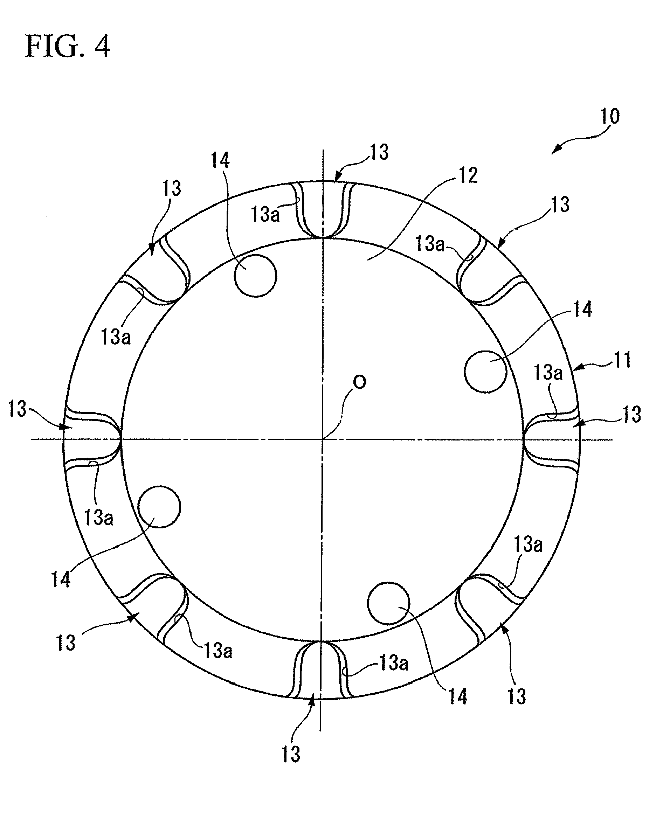

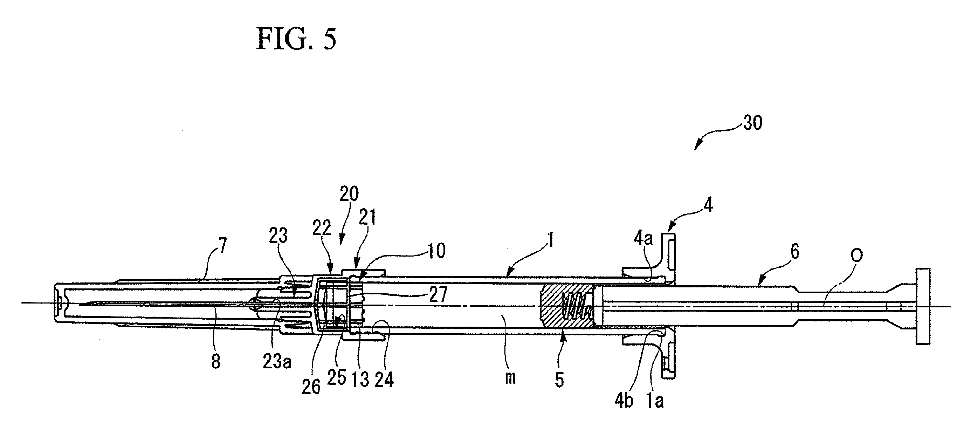

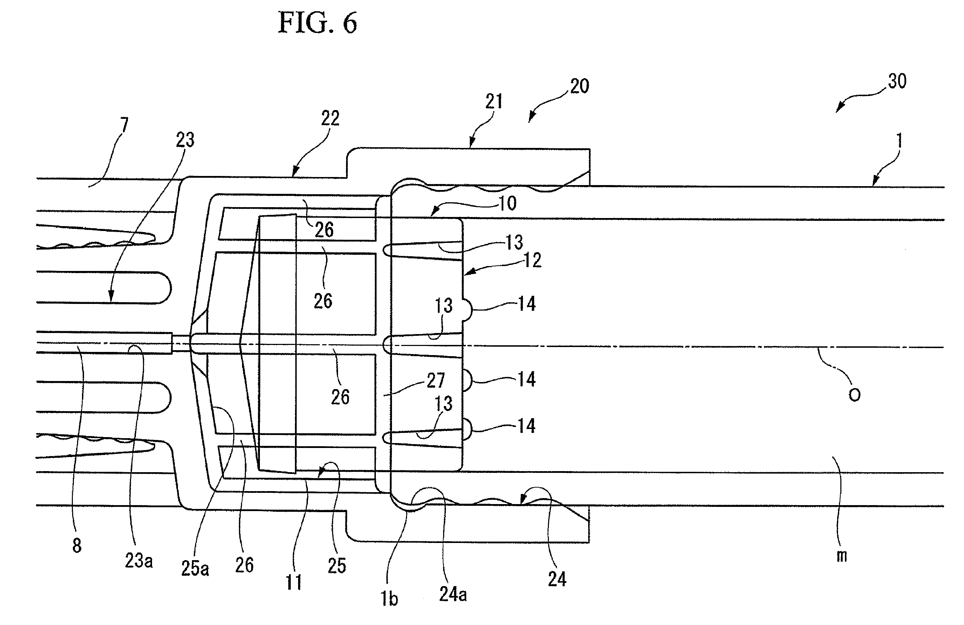

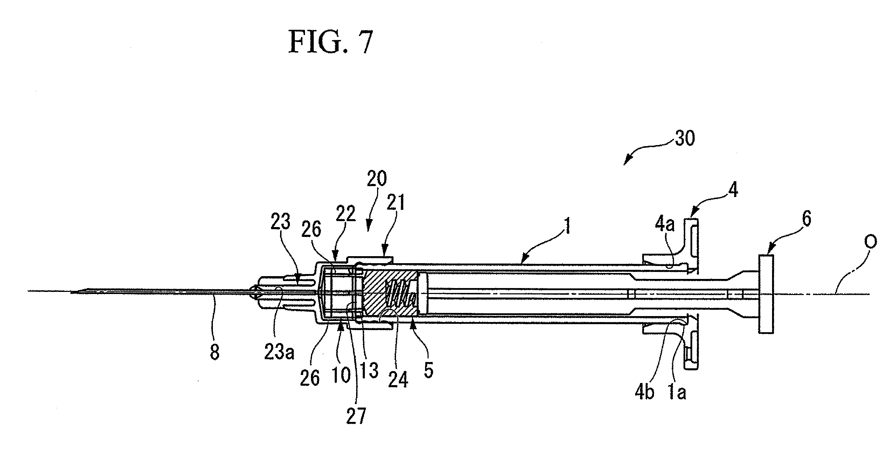

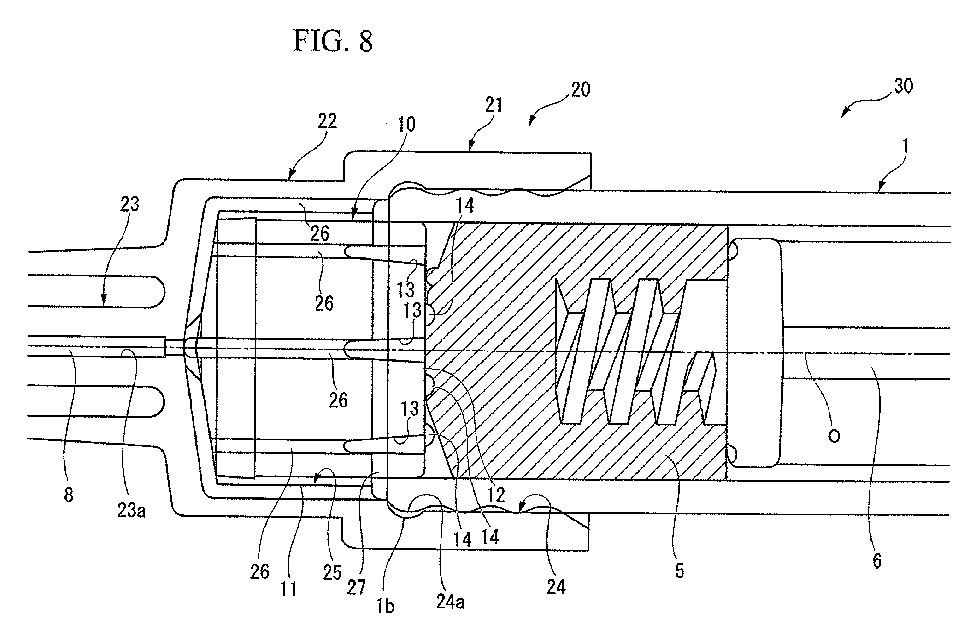

FIG. 1 is a sectional side view showing a combined container-syringe according to one embodiment of the present invention in a state where the combined container-syringe is unused. FIG. 2 is an enlarged view in the vicinity of a front end portion of a glass cartridge of the combined container-syringe shown in FIG 1. FIG 3 is a side view showing a front stopper. FIG. 4 is a rear view showing the front stopper. FIG. 5 is a sectional side view showing the combined container-syringe at the time of an air-eliminating step. FIG. 6 is an enlarged view in the vicinity of the front end portion of the glass cartridge of the combined container-syringe shown in FIG 5. FIG 7 is a sectional side view showing the combined container-syringe after the completion of injection. FIG 8 is an enlarged view in the vicinity of the front end portion of the glass cartridge of the combined container-syringe shown in FIG 7.

As shown in FIG 1, a combined container syringe 30 includes a glass cartridge 1, a needle hub 20 which is attached to a front end portion (left side in FIG. 1, similar in the following description) of the glass cartridge 1, a finger grip 4 which is fitted on an outer circumferential surface of a rear end portion (right side in FIG. 1, similar in the following description) of the glass cartridge 1, a front stopper 10 which is fitted in the front end portion of the glass cartridge 1, a rear stopper 5 which is fitted in the rear end portion of the glass cartridge 1, and a plunger rod 6 which is inserted into the glass cartridge 1 from the rear end side and has a front end portion connected to the rear stopper 5. The finger grip 4 is made of synthetic resin. The front stopper 10 seals the glass cartridge 1 filled with a drug solution m from the front end side in a liquid-tight manner. The rear stopper 5 seals the glass cartridge 1 filled with the drug solution m from the rear end side in a liquid-tight manner. The plunger rod 6 moves the rear stopper 5 forward and backward in a direction of an axis O of the glass cartridge 1.

Furthermore, as shown in FIGS. 3 and 4, the front stopper 10 includes an outer circumferential surface 11, a rear end surface 12, a plurality of air-bubble introducing grooves 13 provided on the outer circumferential surface 11, and a plurality of protrusions (four protrusions in this embodiment) 14 provided on the rear end surface 12 such that the protrusions 14 are uniformly spaced in the circumferential direction. Details of the air-bubble introducing groove 13 will be described later. The protrusions 14 can prevent the close-contact between the stoppers. Therefore, when a plurality of stoppers are washed together, it is possible to prevent insufficient wash of the stoppers due to the close-contact between the stoppers. Furthermore, when a plurality of stoppers are subjected to silicon treatment, it is possible to prevent insufficient applying of silicon to the stoppers due to the close-contact between the stoppers. Furthermore, when one stopper is picked up from a plurality of stoppers housed in a parts feeder and is inserted into a glass cartridge at the time of manufacturing, it is possible to easily pick up only one stopper from the parts feeder.

The glass cartridge 1 is made of transparent glass and has a cylindrical shape extending along the axis O. The glass cartridge 1 is filled with the drug solution m. The front end portion of the glass cartridge 1 is sealed by the front stopper 10 and the rear end portion thereof is sealed by the rear stopper 5.

A ring-shaped protruding portion 1a is provided on the outer circumferential surface of the rear end of the glass cartridge 1. A ring-shaped groove 4b is provided on an inner circumferential surface of a cylindrical hole portion 4a of the finger grip 4. When the ring-shaped protruding portion 1a is fitted in the ring-shaped groove 4b, the finger grip 4 is solidly attached to the glass cartridge 1.

Although the glass cartridge 1 and the finger grip 4 are separately formed and the finger grip 4 is attached to the glass cartridge 1 in this embodiment, the glass cartridge 1 and the finger grip 4 may be formed integrally.

The rear stopper 5 is formed in a substantially cylindrical shape which is coaxial with the axis O and has an outer diameter slightly larger than the inner diameter of the glass cartridge 1. The rear stopper 5 is made of medical rubber (for example, butyl rubber in this embodiment) having corrosion resistance to the drug solution m. When the combined container-syringe 30 is unused, the rear stopper 5 is fitted in the glass cartridge 1 so as to be positioned in the rear end portion of the glass cartridge 1.

The needle hub 20 is made of transparent synthetic resin having moderate rigidity. The needle hub 20 has a cylindrical shape in which a plurality of steps are formed on an outer circumferential surface thereof. The needle hub 20 includes a cylindrical base portion 21, a cylinder portion 22 which is connected to a front end of the base portion 21 in a state where the cylinder portion 22 is reduced in diameter more than the base portion 21 so as to form a step therebetween, and a needle attachment portion 23 which is connected to a front end of the cylinder portion 22 in a state where the needle attachment portion 23 is reduced in diameter more than the cylinder portion 22.

A fitting hole 24 is formed inside the base portion 21 so as to open toward a rear end of the needle hub 20. A bypass chamber 25 is formed inside the cylinder portion 22 which is positioned closer to the front end than the fitting hole 24. The bypass chamber 25 concaves toward the front end.

Furthermore, an introducing hole 23a which is coaxial with the axis O is formed inside the needle attachment portion 23 so as to penetrate the needle attachment portion 23. An injection needle 8 which extends along the axis O toward the front end is attached to the introducing hole 23a in a state where the hole of the injection needle 8 is communicated with the introducing hole 23a.

When the combined container-syringe 30 is unused, a protector 7 for covering the injection needle 8 is attached to the front end portion of the needle hub 20.

The fitting hole 24 is formed for attaching the needle hub 20 to the glass cartridge 1, and has an inner diameter substantially equal to the outer diameter of the glass cartridge 1.

With this constitution, the needle hub 20 can be attached to the front end portion of the glass cartridge 1 by fitting the fitting hole 24 on the front end portion of the glass cartridge 1. A ring-shaped groove 24b around the axis O is formed on an inner circumferential surface of a front end portion of the fitting hole 24. A ring-shaped protruding portion 1b is provided on the outer circumferential surface of the front end of the glass cartridge 1. When attaching the needle hub 20 to the front end portion of the glass cartridge 1, the ring shaped protruding portion 1b is fitted in the ring shaped groove 24a. As a result, the needle hub 20 is solidly attached to the glass cartridge 1.

The bypass chamber 25 is a hole having a bottom portion 25a and has an inner diameter smaller than the inner diameter of the fitting hole 24. A plurality of bypass grooves 26 which extend along the axis O are formed on an inner surface of the bypass chamber 25 such that the bypass grooves 26 are uniformly spaced in the circumferential direction. Each of the bypass grooves 26 extends to the center of the bottom portion 25a of the bypass chamber 25. With this constitution, the bypass grooves 26 are communicated with the introducing hole 23a formed inside the needle attachment portion 23. An annular groove 27 around the axis O is formed at the boundary between the bypass chamber 25 and the fitting hole 24. Rear ends of the bypass grooves 26 are communicated with each other via the annular groove 27.

Similar to the rear stopper 5, the front stopper 10 is formed in a substantially cylindrical shape which is coaxial with the axis O and which has an outer diameter slightly larger than the inner diameter of the glass cartridge 1. The front stopper 10 is made of medical rubber (for example, butyl rubber in this embodiment) having corrosion resistance to the drug solution m. When the combined container-syringe 30 is unused, the front stopper 10 is fitted in the glass cartridge 1 so as to be positioned in the front end portion of the glass cartridge 1.

As specifically shown in FIGS. 3 and 4, the plurality of air-bubble introducing grooves 13 (eight grooves in this embodiment) are formed on the outer circumferential surface 11 of the front stopper 10 such that the air-bubble introducing grooves 13 are uniformly spaced in the circumferential direction. Each of the air-bubble introducing grooves 13 extends from the rear end surface 12 of the front stopper 10 toward the front end of the front stopper 10. That is, opening portions 13a of the air-bubble introducing grooves 13 are formed on the rear end surface 12. As shown in FIG 3, each of the air-bubble introducing grooves 13 extends from the rear end surface 12 of the front stopper 10 to a position crossing over a line k1, that is, a position slightly into a front end side portion 10A from a rear end side portion 10B (i.e., a position on a line k2 in FIG 3). It is preferable that the positions of the air-bubble introducing grooves 13 in the circumferential direction correspond to the positions of the bypass grooves 26.

As shown in FIG 4, each of the opening portions 13a of the air-bubble introducing grooves 13 is formed on the rear end surface 12 so as to extend toward the axis O from an outer circumference of the rear end surface 12 of the front stopper 10.

An explanation will now be made of procedures performed when performing injection to a patient using the combined container-syringe 30 designed as described above. Firstly, the plunger rod 6 of the unused combined container-syringe 30 shown in FIG. 1 is slightly pushed in order to perform an air-eliminating step of eliminating air bubbles remaining in the drug solution m. When the rear stopper 5 connected to the plunger rod 6 is moved forward in the glass cartridge 1, the pressing force by the rear stopper 5 is transmitted to the front stopper 10 via the drug solution m, and the front stopper 10 is moved forward.

As shown in FIGS. 5 and 6, when the front end side portion 10 A (i.e., a portion ranging from the front end to the line k1 shown in FIG 3) of the front stopper 10 enters the bypass chamber 25, the air-bubble introducing grooves 13 of the front stopper 10 come to be slightly communicated with the annular groove 27 of the bypass chamber 25, and the tight-sealing of the front end side of the glass cartridge 1 which is filled with the drug solution m is released.

In this state, air bubbles introduced in the air-bubble introducing grooves 13 pass through the annular groove 27, the bypass grooves 26, and the introducing hole 23a to be eliminated outside of the combined container-syringe 30 via the front end of the injection needle 8. It is possible to effectively eliminate air bubbles in the drug solution m by performing the above-described air-eliminating step.

After the air-eliminating step, the injection needle 8 is inserted into a desired site of the patient, and the plunger rod 6 is further pushed so that the drug solution m in the glass cartridge 1 is administered to the inside of the patient's body after passing through the air-bubble introducing grooves 13, the annular groove 27, the bypass grooves 26, the introducing hole 23a, and the injection needle 8. As shown in FIGS. 7 and 8, when the plunger rod 6 is completely pushed, the rear stopper 5 comes into contact with the front stopper 10, and the drug solution m in the glass cartridge 1 is completely injected into the patient's body without remaining in the glass cartridge 1.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

## Claims

1. A combined container-syringe comprising:
a cartridge;
a needle hub having a base portion which is fitted on an outer circumferential surface of a front end portion of the cartridge, a cylinder portion which is connected to a front end of the base portion and inside which a bypass chamber is formed, and a needle attachment portion which is connected to a front end of the cylinder portion and inside which an introducing hole communicated with an injection needle is formed;
a front stopper which is fitted in the front end portion of the cartridge in a liquid-tight manner;
a rear stopper which is fitted in a rear end portion of the cartridge in a liquid-tight manner; and
a plunger rod which is connected to the rear stopper,
wherein: a bypass groove which extends along an axis of the cartridge and is communicated with the introducing hole, and an annular groove which is connected to a rear end of the bypass groove are formed on an inner circumferential surface of the bypass chamber;
an air-bubble introducing groove is formed on an outer circumferential surface of the front stopper, the air-bubble introducing groove extending from a rear end surface of the front stopper toward a front end; and
the air-bubble introducing groove and the annular groove are communicated with each other in a state where a part of the front stopper is disposed in the cartridge.

2. The combined container-syringe according to claim 1,
wherein the air-bubble introducing groove extends, on the rear end surface of the front stopper, from an outer circumference of the rear end surface of the front stopper toward the axis of the cartridge.

3. The combined container-syringe according to claim 1,
wherein a plurality of the bypass grooves are formed on the inner circumferential surface of the bypass chamber such that the bypass grooves are uniformly spaced in a circumferential direction of the inner circumferential surface of the bypass chamber; and
a plurality of the air-bubble introducing grooves are formed on the outer circumferential surface of the front stopper such that the air-bubble introducing grooves are uniformly spaced in a circumferential direction of the outer circumferential surface of the front stopper.

4. The combined container-syringe according to claim 3,
wherein the positions of the plurality of the air-bubble introducing grooves in the circumferential direction correspond to the positions of the plurality of the bypass grooves.
